# EUROPEAN PATENT SPECIFICATION

(11) **EP 1 417 984 B1**
(45) Date of publication and mention of the grant of the patent: **12.03.2008**
(21) Application number: 03078506.7
(22) Date of filing: 07.11.2003
(51) Int. Cl.: A61F 2/06, A61M 25/10

(54) **Balloon catheter**
Ballonkatheter
Cathéter à ballonnet

(30) Priority: 08.11.2002 US 290603
(43) Date of publication of application: 12.05.2004
(73) Proprietor: Blue Medical Devices B.V., 5708 HN Helmond (NL)
(72) Inventor: Meens, Hendrik Jozef Maria, 6002 WH Weert (NL); Horvers, Ronald Adrianus Maria, 5663 PH Geldrop (NL)
(74) Representative: Brants, Johan P.E.

(56) References cited:
- EP-A- 0 611 582
- EP-A- 0 834 293
- EP-A- 1 256 357
- EP-A- 1 270 040
- WO-A-99/25417
- US-A- 5 141 494
- US-A- 5 409 458
- US-A- 5 545 132
- US-A- 5 549 556
- US-A- 5 575 771
- US-A- 5 980 484
- US-A- 6 007 517
- US-A- 6 010 480

## Description

### Technical Field

The present invention relates to a balloon catheter comprising a catheter tube, a tip, a stiffening wire connecting the catheter tube to the tip, an inflatable balloon which is attached to the catheter tube, and guide wire receiving lumens.

### Background Information

Balloon catheters are known, for example, from the following United States Patents: No. 4,762,129, granted August 9, 1988 to Tassilo Bonzel; No. 5,496,346, granted March 5, 1996 to Michael J. Horzewski and Paul G. Yock; and No. 6,071,285, granted June 6, 2000, to Robert D. Lashinski, Dennis L. Brooks, Philip J. Haarstad, and Geoffrey A. Orth. A balloon catheter according to the preamble of claim 1 is known from US-A- 5 549 556. There is a need for a balloon catheter that is easy to install on a guide wire that is used to guide its movement inside a patient. A principal object of this invention is to provide such a balloon catheter.

### Brief summary of the Invention

Balloon catheters of the present invention are basically characterized by a catheter tube having a balloon end and a tip that is spaced from the balloon end of the catheter tube. A stiffening wire extends between and interconnects the balloon end of the catheter tube and the tip. An inflatable balloon is positioned between the balloon end of the catheter tube and the tip. The balloon is connected to the balloon end of the catheter tube and to the tip. The catheter tube includes a flexible tube having a first end connected to the catheter tube and a second end that includes a lumen. In use, a guide wire extends through the catheter tube and the flexible tube and into and through the tip, along side of the balloon. When the balloon is deflated, the flexible tube is generally straight and is along side of the balloon. When the balloon is inflated, the flexible tube is curved and bends around the balloon.

In preferred form, the tip also includes a flexible tube that extends from the tip towards the catheter tube and has a lumen at its end. The guide wire extends through the catheter tube, through both of the flexible tubes, and through the tip.

In preferred form, the outside surface of the balloon has at least one helically shaped groove formed in it for creating flexibility in a direction transverse to the balloon. Preferably, the outside surface of the balloon has a first helical groove extending around the balloon in a first direction and a second helical groove extending around the balloon in the opposite direction.

The invention includes balloon catheters of the several types disclosed herein to which a single flexible tube with a lumen at its end or two flexible tubes, each with a lumen at its end, have been added.

Other objects, advantages and features of the invention will become apparent from the description of the best mode set forth below, from the drawings, from the claims and from the principles that are embodied and the specific structures that are illustrated and described.

### Brief Description of the Drawings

In the drawings, like element designations refer to like parts throughout, and:
Fig. 1 is a pictorial view of a balloon catheter in an inflated condition;
Fig. 2 shows the balloon catheter of Fig. 1 in a non-inflated condition;
Fig. 3 is a sectional view of a balloon comprising a stiffening wire and a guide wire, which illustrates the manner in which the balloon catheter of Fig. 1 is obtained by means of a first folding method;
Fig. 4 is a sectional view like Fig. 3, but showing a second folding method;
Fig. 5 is a side view of another balloon catheter in an uninflated state;
Fig. 6 is a side view of the catheter in Fig. 5 in an inflated state;
Fig. 7 is a side view of still another balloon catheter during the execution of the method;
Fig. 8 is a side view of the final product of Fig. 7;
Fig. 9 is a side view of yet still another balloon catheter in an uninflated state;
Fig. 10 is a side view of the catheter in Fig. 9 in an inflated state;
Fig. 11 is a side view of yet still another balloon catheter in an uninflated state;
Fig. 12 is a side view of the catheter of Fig. 11 in an inflated state;
Fig. 13 is a side elevational view of a balloon catheter according to the invention and of a type shown by Figs. 1 and 2, showing a tip at the right end thereof for receiving a guide wire such as shown in Fig. 1; and
Fig. 14 is a view like Fig. 13 in which the tip comprises a flexible tube including a lumen.

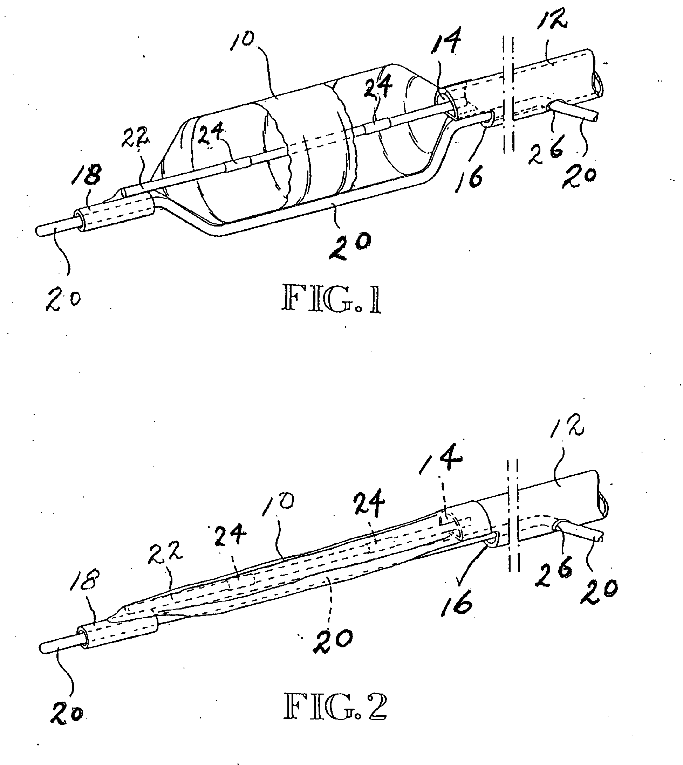

The pictorial view of Fig. 1 shows a balloon catheter with the balloon 10 in an inflated condition. The balloon catheter comprises a catheter tube 12 having a first lumen 14 and a second lumen 16, each lumen 14, 16 having a proximal end and a distal end. The distal end of the first lumen 14 opens into the balloon 10. The inflatable balloon 10 likewise has a proximal end and a distal end, the proximal end being fixed to the catheter tube 12 and the distal end being fixed to a tip 18. The balloon catheter furthermore comprises a guide wire 20 to be received in the second lumen 16 and a stiffening wire 22 received within the first lumen 14. The stiffening wire 22 is provided with marking bands 24, which are discernible on x-ray equipment. The stiffening wire 22 extends from the first lumen 14, through the open distal end thereof and through the balloon 10, into the distal end of said balloon 10. This stiffening wire 22 is fixed to the tip 18 together with the distal end of the balloon 10.

The distal end of the second lumen 16 is open, and it is positioned proximally of the proximal end of the balloon 10. An opening 26, which is in communication with the second lumen 16, is furthermore provided in the catheter tube 12. Said opening 26 is positioned proximally of the distal end of the second lumen 16. The guide wire 20 can be passed through the opening 26 in the catheter tube 12 into the second lumen 16, and exit the second lumen 16 through the open distal end thereof. The guide wire 20 extends outside of the balloon 10 and is passed through the lumen of the tip 18.

In an inflated condition of the balloon 10 (Fig. 1), the guide wire 20 is positioned free of and outside of the balloon 10 between the open distal end of the second lumen 16 and the tip 18. In a non-inflated condition of the balloon 10 (Fig. 2), the guide wire 20 is still positioned outside of the balloon 10, but it is received within a cavity that has been obtained by folding the balloon 10 according to a special folding method.

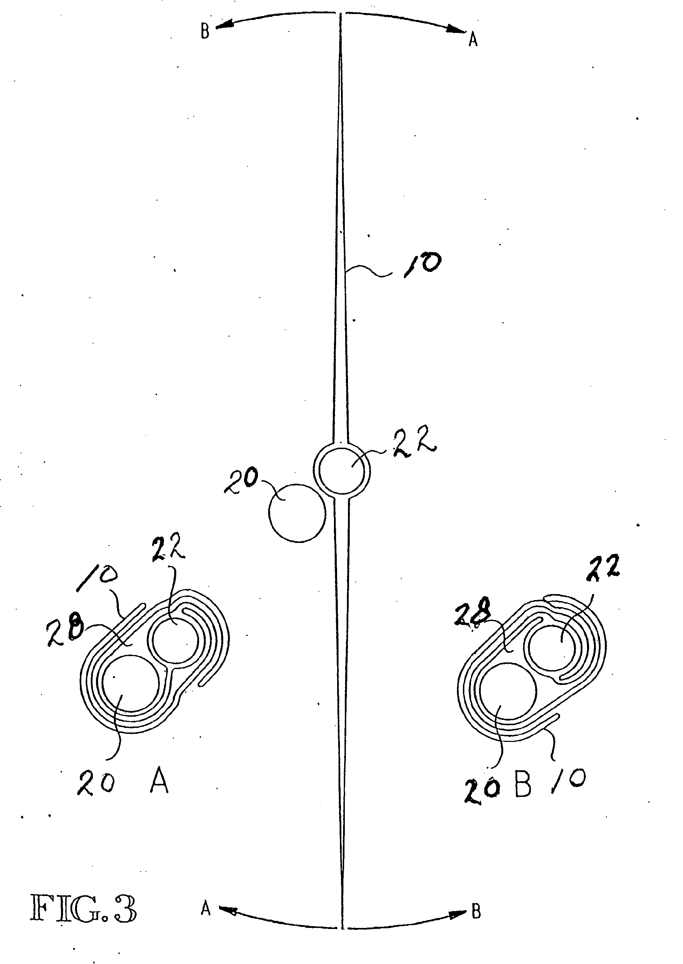

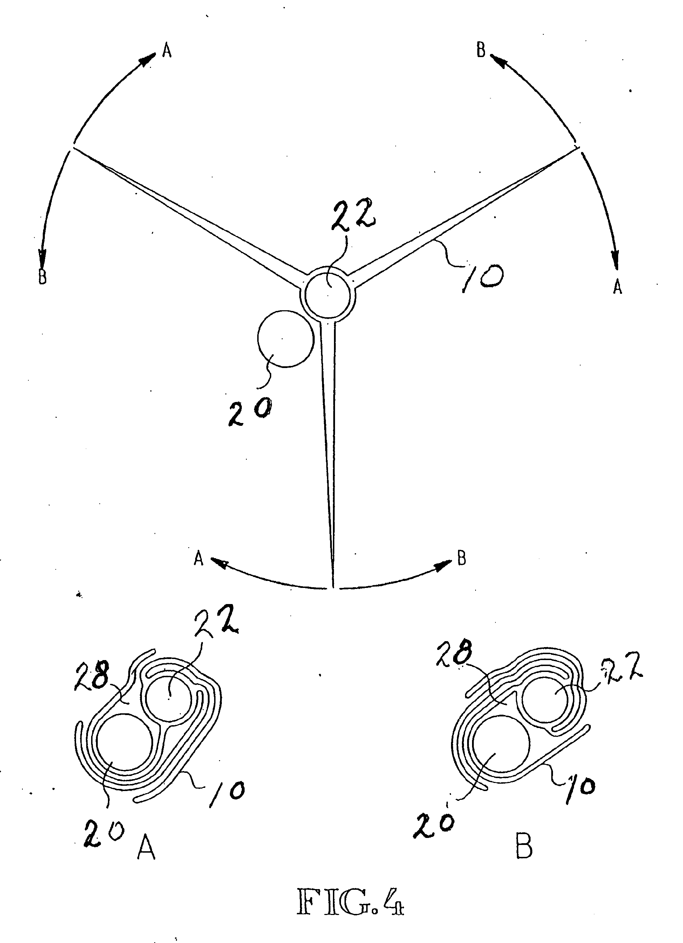

The folding method is illustrated in Figs. 3 and 4. In Figs. 3 and 4, the same reference numerals are used as in Figs. 1 and 2. A balloon 10 with the stiffening wire 22 received therein is folded, in a manner which is known per se, to form a kind of propeller profile comprising two (Fig. 3), three (Fig. 4) or four (not shown) wings, depending on the diameter of the balloon 10. The guide wire 20 is arranged parallel to the stiffening wire 22. Finally, the wings are folded in anti-clockwise direction, indicated at B, or clockwise direction, indicated at A, to obtain the result indicated at A or the result indicated at B, respectively. The sectional views of Figs. 3 and 4 clearly show at A and B that as a result of the special manner of folding the balloon 10, the guide wire 20 is received in a cavity 28 that has been obtained by folding the wings of the balloon 10 around the stiffening wire 22 and the guide wire 20. As a result, a very compact structure is obtained, which makes it possible to guide the balloon catheter through vessels and lumina.

It is noted that it is possible to have the guide wire 6 extend entirely through the second lumen 4 to the proximal end thereof.

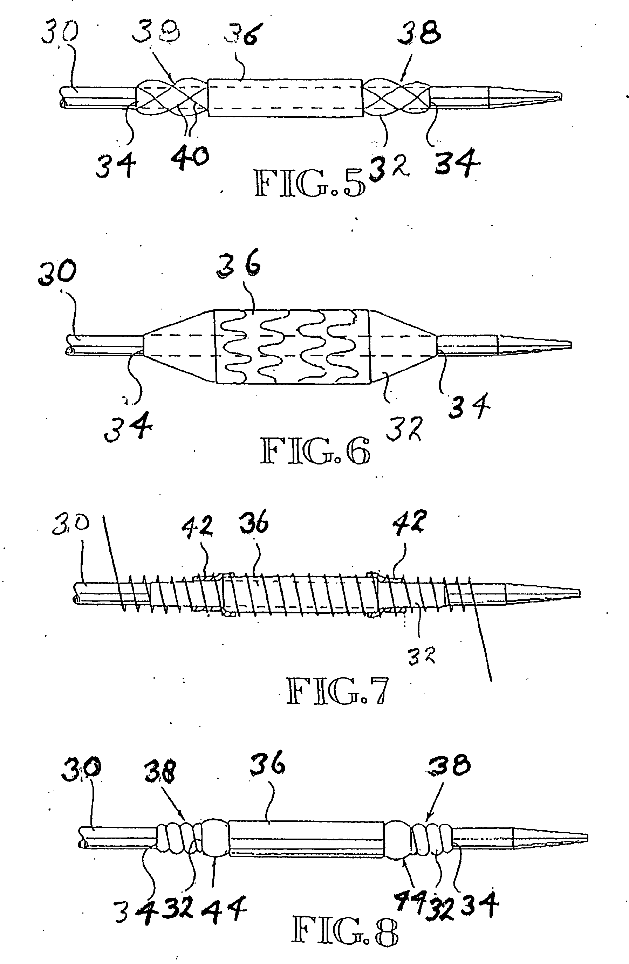

The balloon catheter shown in Pigs. 5 and 6 comprises a catheter tube 30, an inflatable balloon 32, which at its ends 34 is attached to the catheter tube 30, and a stent 30 attached around the balloon 32. The stent 36 extends partly along the length of the balloon 32 such that with the catheter, both distally and proximally next to the stent 36, balloon material lies freely. In an uninflated state (Fig. 5), the outside surface of the balloon 32 has a relief structure 38 that in the inflated state has virtually or completely disappeared (Fig. 6). The relief structure 38 gives the distal end of the catheter its required flexibility.

In the embodiments shown by Pigs. 5-8, the relief structure 38 consists of two grooves 40, which extend helically from one end 34 to the other end 34 of the balloon 32 and across each other. The uninflated balloon 32 (Fig. 5) has thereby obtained a padded relief surface. It is also possible to provide a relief structure with a single helically shaped groove 5 whereby the uninflated balloon 32 displays a relief surface in the form of a helix. Other relief structures are of course possible, provided that the relief structure on the catheter creates the necessary flexibility in a direction transverse to the longitudinal direction of the balloon.

One way of obtaining the relief structure as shown in the drawings is by winding a wire helically around the balloon 32. This wire may, for example, be nylon wire or wire made of a different material that contracts somewhat on heating. After the wire has been wound around the balloon, a sleeve is pulled over the balloon. Subsequently, with the application of raised pressure to the inside of the balloon, the balloon is heated in such a way that in an uninflated state the balloon obtains a relief structure that on dilating of the balloon at the dilatation site in the vessel or lumen will virtually or completely disappear. The sleeve is then removed and the balloon catheter can be inserted into a vessel or lumen.

The method can be carried out in two ways. In the first way, the profile is first applied to the balloon by winding this around with wire and heating it under internal pressure, and then the stent is placed on the balloon. This results in improved flexibility and maneuverability of the distal part of the catheter. Better fixing of the stent on the "rough", profiled surface of the balloon is also achieved. In the second way, a stent is first placed on the balloon and then the complete distal part of the catheter, namely the balloon including the stent that has been placed in position, is wound around with wire, preferably nylon wire. Then the whole assembly is heated at a certain internal pressure. Winding produces a helical profile in the balloon material next to the stent. At the same time the diameter of the catheter with the stent is reduced because the nylon wire shrinks on heating. In a variation of this last method, first filler material 42 is placed next to the stent 36, over which wire is then also wound (see Fig. 7). In the end this results in a small increase 44 in the balloon material next to the stent 36 (see Fig. 8), into which the ends of the stent 36 can be pressed. As a result the ends of the stent 36 are better protected if the stent placing system is passed in a curved trajectory.

Instead of winding the wire, the balloon may be placed in a mould, which is provided with the relief pattern required, in order for it to obtain, under raised pressure and temperature, the relief structure required.

Before the balloon is provided with its relief structure, preferably it is folded in the usual way in order to reduce its profile. By applying the relief structure, the profile will be reduced as an additional, advantageous effect.

Note that, although it is not shown in the drawings, it is possible to provide the outside surface of the balloon with various helical grooves that cross each other. In addition to helical grooves, grooves of other shapes are also possible.

The balloon catheter may be used not only in cardiological but also radiological interventions.

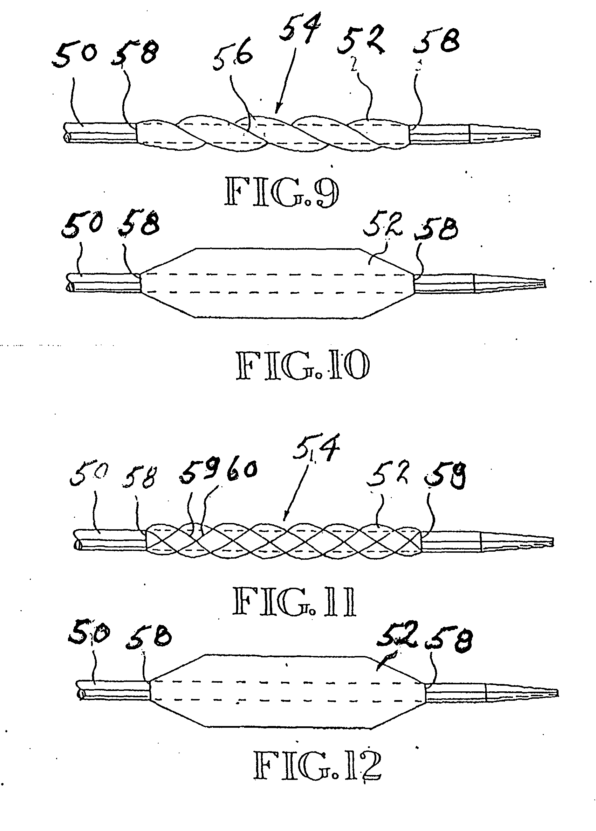

The balloon catheter shown by Figs. 9-12 comprises a catheter tube 50 and an inflatable balloon 52, which at its ends is attached to the catheter tube 50. In an uninflated state (Figs. 9 and 11), the outside surface of balloon 52 has a relief structure 54 that in the inflated state has virtually or completely disappeared (Figs. 10 and 12). The relief structure gives the catheter its required flexibility.

In the first embodiment according to Figs. 9 and 10, the relief structure 54 consists of one groove 56, which extends helically from one end 58 to the other end 58 of the balloon 52, over the outside surface thereof. The uninflated balloon 52 has thereby obtained a helical relief surface. In the second embodiment according to Figs. 11 and 12, the relief structure 54 consists of two grooves 56, 58,which extend helically from one end 58 to the other end 58 of the balloon 52 and thereby cross each other. The uninflated balloon 52 has hereby obtained a padded relief surface.

Other relief structures are of course possible, provided that the relief structure on the catheter creates the necessary flexibility in a direction transverse to the longitudinal direction of the balloon.

One way of obtaining the relief structure as shown in the drawings is by winding a wire helically around the balloon 52. If the wire is wound only in the forward direction, the structure according to Fig. 9 is obtained, and if the wire is also wound in the return direction, the structure in Fig. 11 is obtained. After the wire has been wound around the balloon, a sleeve is pulled over the balloon. Subsequently, with the application of raised pressure to the inside of the balloon, the balloon is heated in such a way that, in an uninflated state, the balloon obtains a relief structure that on dilating of the balloon at the dilatation site in the vessel or lumen will virtually or completely disappear. The sleeve is then removed and the balloon catheter can be inserted into a vessel or a lumen

Instead of winding a wire, the balloon may be placed in a mould, which is provided with the relief pattern required in order for it to obtain, under raised pressure and temperature, the relief structure required.

Before the balloon is provided with its relief structure, preferably it is folded in the usual way in order to reduce its profile. By applying the relief structure, the profile will be reduced still further as an additional, advantageous effect.

Note that, although it is not shown in the drawings, it is possible to provide the outside surface of the balloon with various helical grooves that cross each other.

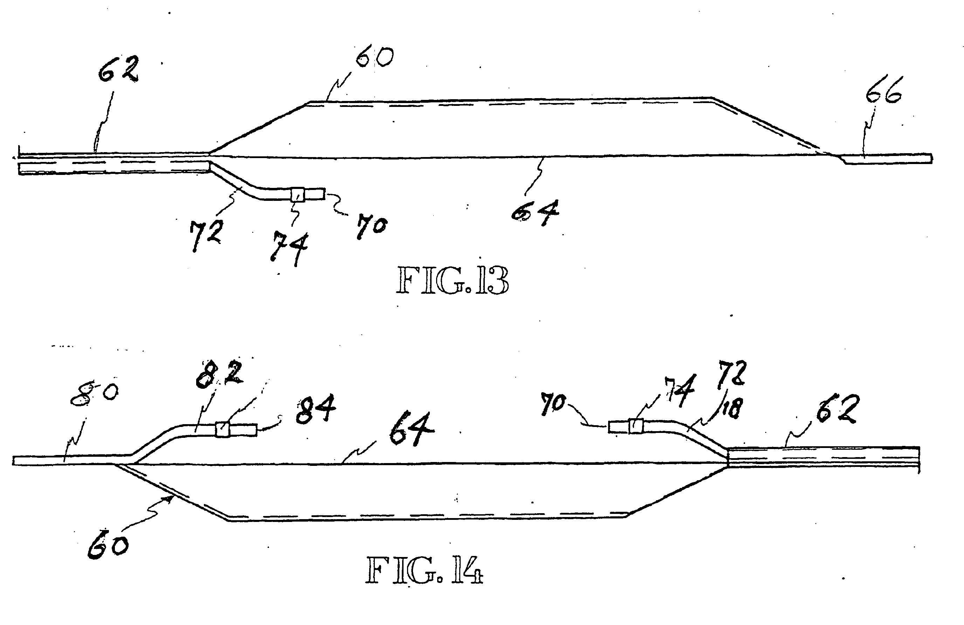

Figs. 13 and 14 show a modified form of the catheter that is shown in Figs. 1-4. A balloon 60 is attached to a catheter tube 62. A stiffening wire 64 is used which includes a tip 66 at the end thereof opposite the catheter tube 62. The tip 66 receives a guide wire 6 (in Fig. 1). Lumen 70 is elongated and is at an end of a flexible tube 72. When the balloon 60 is deflated, the tube 72 is straight. When the balloon 60 is inflated, the tube 72 bends around balloon 60 as shown in Fig. 1. The tube 72 includes a marker 74.

The embodiment of Fig. 14 also includes a tube 80 having a flexible tube section 82 and a lumen 84 for receiving a guide wire 6 (Figs. 1 and 2). The marker 86 can be applied on the stiffening wire 88 or on the flexible tubes 90, 82. The elongation of lumens 92 and 84 by flexible tubes 90, 82 improves the ease of use when applying the balloon catheter on a guide wire. In use, the guide wire 6 is placed inside a patient. Secondly, the balloon catheter is installed on the guide wire 6 so that the guide wire 6 can be used to guide its movement. The ease of applying the balloon catheter on the guide wire 6 is an important factor to a surgeon who is choosing which type of balloon catheter to use. The balloon catheter of the invention is relatively easy to use. The balloon catheter disclosed by the aforementioned U.S. Patent 071,285 will prove difficult to apply onto a guide wire.

A balloon catheter constructed according to either Fig. 13 or either Fig. 14 will undergo two heat treatments. The first heat treatment for folding the balloon is described in U.S. Application Serial No. 10/117.922.38 (US-A-2003/0191436). See Fig. 4 of that publication and Fig. 4 of this application. The second heat treatment is a part of the checker technique that is disclosed in U.S. Application Serial Nos. 10/140, 524 (US-A-2003/0014070) and 10/140, 479 (US-A-2003/0014100). When the checker technique is used, a groove will be formed in the balloon for receiving the guide wire. As a result, the balloon catheter can be easily applied on the guide wire. This groove resembles a cavity along the length of the balloon that remains after the balloon is removed from the guide wire 6 after the second heat treatment. Because of the presence of the groove, the balloon catheter of the present invention can equally be used without a stent. This is in contrast to the balloon catheter disclosed by the aforementioned U.S. Patent No. 6,071,285. The Lashinski balloon catheter requires a stent for holding the guide wire against the balloon. A stent is also necessary when the balloon passes a corner in a blood vessel. The illustrated embodiments are only example of the present invention and, therefore, are non-limitative. It is to be understood that many changes in the particular structure, materials and features of the embodiments may be made without departing from the scope of the invention being defined in the claims.

## Claims

1. A balloon catheter, comprising:
a catheter tube (62) having a balloon end;
a tip (66) spaced from the balloon end of the catheter tube (62);
a stiffening wire extending between and interconnecting the balloon end of the catheter tube (62) and the tip (66);
an inflatable balloon (60) positioned between the balloon end of the catheter and the tip (66), the balloon (60) being connected to the balloon end of the catheter tube (62) and to the tip (66), **characterized in that**
the catheter tube (62) includes a flexible tube (72) having a first end connected to the balloon end of the catheter tube (62) and a second end positioned between the catheter tube (62) and the tip (66) and including a lumen (70),
wherein in use a guide wire (6) extends through the catheter tube (62) and the flexible tube (72) and into and through the tip (66) along side the balloon (60), and wherein the flexible tube (72) bends around the balloon (60) when it is inflated.

2. The balloon catheter of claim 1, wherein the tip (66) also includes a flexible tube (82) having one end connected to the tip (66) and the opposite end including a lumen (84) for receiving the guide wire (6).

3. The balloon catheter of claim 1 or 2, wherein the balloon (60) is inflated to move it from a deflated condition to an inflated condition, and the flexible tube (72; 82) is generally straight and is along side the balloon (60) when the balloon (60) is deflated and is curved and bends around the balloon (60) when the balloon (60) is inflated.

4. The balloon catheter of one of the preceding claims, wherein the balloon (60) has an outside surface and at least one helically shaped groove formed in said surface for creating flexibility in a direction transverse to the balloon (60).

5. The balloon catheter of one of the claims 1-4, wherein the balloon (60) has a sidewall that includes a first helical groove extending around the balloon (60) in a first direction and a second helical groove extending around the balloon (60) in a second direction, so as to create flexibility in the balloon (60) in a direction transverse to the balloon (60).

## Patentansprüche

1. Ballonkatheter, umfassend:
einen Katheterschlauch (62),der ein Ballonende aufweist;
eine Spitze (66), die von dem Ballonende des Katheterschlauchs (62) räumlich getrennt ist;
einen Versteifungsdraht, der sich zwischen dem Ballonende des Katheterschlauchs (62) und der Spitze (66) erstreckt und beide miteinander verbindet;
ein aufblasbarer Ballon (60), der zwischen dem Ballonende von dem Katheter und der Spitze (66) positioniert ist, wobei der Ballon (60), der mit dem Ballonende von dem Katheterschlauch (62) und der Spitze (66) verbunden ist,
**dadurch gekennzeichnet ist, dass**
der Katheterschlauch (62) einen biegsamen Schlauch (72) enthält, der ein erstes Ende aufweist, welches mit dem Ballonende von dem Katheterschlauch (62) verbunden ist, und ein zweites Ende, welches zwischen dem Katheterschlauch (62) und der Spitze (66) positioniert ist und ein Lumen (70) aufweist,
wobei während der Verwendung ein Führungsdraht (6) sich durch den Katheterschlauch (62) und den biegsamen Schlauch (72) in und durch die Spitze (66) längsseits von dem Ballon (60) erstreckt und wobei der biegsame Schlauch (72) sich um den Ballon (60) biegt, wenn dieser aufgeblasen ist.

2. Ballonkatheter nach Anspruch 1, wobei die Spitze (66) ebenfalls einen biegsamen Schlauch (82) einbezieht, von dem ein Ende mit der Spitze (66) verbunden ist und das gegenüberliegende Ende ein Lumen (84) zum Aufnehmen des Führungsdrahtes (6) einschließt.

3. Ballonkatheter nach Anspruch 1 oder 2, wobei der Ballon (60) aufgeblasen ist, um ihn aus einem unbefüllten Zustand in einen aufgeblasenen Zustand zu versetzen, und der biegsame Schlauch (72, 82) im Wesentlichen gerade und längsseits von dem Ballon (60) ist, wenn der Ballon unbefüllt ist, und gekrümmt ist und sich rund um den Ballon (60) biegt, wenn der Ballon (60) aufgeblasen ist.

4. Ballonkatheter gemäß einem der vorstehenden Ansprüche, wobei der Ballon (60) eine äußere Oberfläche und mindestens eine schraubenartig geformte Furche in der Oberfläche aufweist, die zur Herstellung der Biegsamkeit in einer Richtung dient, die quer zu dem Ballon (60) verläuft.

5. Ballonkatheter nach einem der Ansprüche 1 - 4, wobei der Ballon (60) eine Seitenwand aufweist, die eine erste schraubenartige Furche beinhaltet, die sich rund um den Ballon (60) in einer ersten Richtung erstreckt und eine zweite schraubenartige Furche, die sich rund um den Ballon (60) in einer zweiten Richtung derart erstreckt, sodass damit eine Biegsamkeit in dem Ballon (60) hergestellt wird, die in einer Richtung quer zu dem Ballon (60) verläuft.

## Revendications

1. Cathéter-ballon, comprenant :
un tube de cathéter (62) ayant une extrémité-ballon ;
un embout (66) espacé de l'extrémité-ballon du tube de cathéter (62) ;
un fil de raidissement s'étendant entre et interconnectant l'extrémité-ballon du tube de cathéter (62) et l'embout (66) ;
un ballon gonflable (60) positionné entre l'extrémité-ballon du cathéter et l'embout (66), le ballon (60) étant connecté à l'extrémité-ballon du tube de cathéter (62) et à l'embout (66),
**caractérisé en ce que**
le tube de cathéter (62) comprend un tube flexible (72) ayant une première extrémité connectée à l'extrémité-ballon du tube de cathéter (62) et une deuxième extrémité positionnée entre le tube de cathéter (62) et l'embout (66) et comprenant un lumen (70),
dans lequel, en utilisation, un fil de guidage (6) s'étend à travers le tube de cathéter (62) et le tube flexible (72) et dans et à travers l'embout (66) à côté du ballon (60), et dans lequel le tube flexible (72) se plie autour du ballon (60) lorsqu'il est gonflé.

2. Cathéter-ballon selon la revendication 1, dans lequel l'embout (66) comprend également un tube flexible (82) ayant une extrémité connectée à l'embout (66) et l'extrémité opposée comprenant un lumen (84) pour recevoir le fil de guidage (6).

3. Cathéter-ballon selon la revendication 1 ou la revendication 2, dans lequel le ballon (60) est gonflé pour le faire passer d'un état dégonflé à un état gonflé, et le tube flexible (72 ; 82) est généralement droit et se trouve à côté du ballon (60) lorsque le ballon (60) est dégonflé et est incurvé et se plie autour du ballon (60) lorsque le ballon (60) est gonflé.

4. Cathéter-ballon selon l'une quelconque des revendications précédentes, dans lequel le ballon (60) a une surface extérieure et au moins une gorge de forme hélicoïdale formée dans ladite surface pour créer la flexibilité dans une direction transversale au ballon (60).

5. Cathéter-ballon selon l'une quelconque des revendications 1 à 4, dans lequel le ballon (60) a une paroi latérale qui comprend une première gorge hélicoïdale s'étendant autour du ballon (60) dans une première direction et une deuxième gorge hélicoïdale s'étendant autour du ballon (60) dans une deuxième direction, de manière à créer la flexibilité dans le ballon (60) dans une direction transversale au ballon (60).
